# EUROPEAN PATENT SPECIFICATION

(11) **EP 2 039 364 B1**
(45) Date of publication and mention of the grant of the patent: **10.10.2012**
(21) Application number: 07794043.5
(22) Date of filing: 08.06.2007
(51) Int. Cl.: A61K 33/00, A61P 15/10, A61K 45/06

(54) **MEANS AND METHOD FOR ENHANCING A HUMAN SEXUAL ACTIVITY**
MITTEL UND VERFAHREN ZUR ERHÖHUNG DER SEXUELLEN AKTIVITÄT EINES MENSCHEN
AGENT ET PROCÉDÉ FAVORISANT L'AUGMENTATION DE L'ACTIVITÉ SEXUELLE D'UN ÊTRE HUMAIN

(30) Priority: 19.06.2006 RU 2006121369
(43) Date of publication of application: 25.03.2009
(73) Proprietor: Woodford Associates Limited, London W4 3HR (GB); Soloviev, Sergey Pavlovich, Moscow 115432 (RU)
(72) Inventor: SOLOVIEV, Sergey Pavlovich, Moscow, 115432 (RU)
(74) Representative: Hayes, Adrian Chetwynd
(86) International application number: PCT/RU2007/000309
(87) International publication number: WO 2008/002191

(56) References cited:
- WO-A-2005/065631
- WO-A-2005/070438
- WO-A-2006/085785
- WO-A-2006/098650
- JP-A- 2004 075 654
- JP-A- 2004 175 776
- JP-A- 2004 315 487
- RU-C1- 2 101 982
- RU-C1- 2 146 940
- RU-C1- 2 241 466
- RU-C1- 2 270 017

## Description

The invention refers to medicine, in particular, andrology, and relates to a means and method to enhance a human sexual activity.

Problems with potency are now essential for many men of different ages.

Potency can be defined as a desire (on the basis of sexual activity-libido) to perform coitus combined with a physical ability to realize it (an appropriate erection available). In general, combination of the elements mentioned above forms a human sexual activity.

Erectile dysfunction is a medical term synonymous with impotency. The term proposed by the US National Institute of Health and adopted by international organizations of urologists and andrologists in 1992 includes both problems with erection and orgasm disturbance, as well as libido weakening. More than 150 mln. men worldwide suffer from erectile dysfunction and in 20 years they can reach 322 mln. In Russia every third man, who is past forty, occasionally faces with potency problems (Kamalov A.A. et al. "Consilium Medicum", Vol. 06/No.5/2004).

The sexual activity decreased (due to age or not associated with age) is a painful issue for most men. Many men suffer from some degree of impotency resulting from psychological and/or physiological reasons often interrelated. The basis of the reasons can be neurogenic disorders, vascular dystonia appearing as a result of a general trend for decreased physical activity and increased psychological stresses of a human being of the time, as well as side effects of medicinal means intake, bad habits, etc.

Impotency, decreased sexual activity and potentiality of men has recently been considered as a disease of civilization. The overall quality of life of an individual is substantially determined by the quality of its sexual life, so the search of acceptable means and methods to improve it is topical.

In severe cases of impotency caused, for example, by paralysis of motor nerves activating an erection, surgical treatment is used. For the purpose of erection normalization (improvement) psychotherapeutic methods are used, as well as a number of medicinal means of different groups in the form of injections, pills, creams or ointments (psychostimulants, antidepressants, adrenergic agonists, androgenic hormones, vasoactive substances, etc.). But despite a great progress in this area, increasing a range of means to normalize the problem remains very topical. Quite often, the above mentioned means cause marked side effects. Since the result of application of any medicinal means by every person is very individual, the range expanded increases for each consumer an opportunity to choose the most suitable means. On the other hand, it is desirable for the means mentioned above to be multipurpose and suitable for a large number of consumers who need such a medicinal means.

RU2146940 discloses the treatment of erectile dysfunction with a homeopathic preparation of several herbal extracts. RU2241466 discloses the treatment of male climacteric syndrome with an infusion of ozonised physiological solution.

The natural water is known to be a composition of nine isotope types of water molecules, in particular, ¹H ¹⁶O, ¹H₂ ¹⁷O, ¹H ¹⁸O, ¹H²H¹⁶O, ¹H²H¹⁷O, ¹H²H¹⁸O, ²H₂¹⁶O, ²H₂¹⁷O, ²H₂¹⁸O, while the number of the lighter molecules ¹H₂¹⁶O is about 99.7317% (Vienna Standard Mean Ocean Water, VSMOW), while the total number of the remaining eight heavy types of water molecules is around 0.2683% (0.199983% ¹H₂¹⁸O, 0.0372% ¹H₂¹⁷O, 0.031069% ¹H²H ¹⁶O, 0.0000623% ¹H²H ¹⁸O, and 0.0000116% ¹H²H ¹⁷O according to Rothman et al., J. Quant. Spectrosc. Radiat. Transfer, 1998, 60, 665; Rothman et al., J. Quant. Spectrosc. Radiat. Transfer, 2003, 82, p.9).

In natural waters, occurrence of isotope types of H₂O molecules depends on the geographical location of the region, climatic conditions and the season [Ferronsky V.I., Polyakov VA "Hydrosphere Isotopy", Publishing Hose "Science", 1983, p. 47, p. 10, 47, 46, 10]. The ratio of heavy isotopes is usually expressed through a deviation δ from standard VSMOW. Ocean water, which is the main part of water on the Earth, contains less ¹H₂¹⁶O compared with the base reserves of fresh water. For freshwater sources, this figure varies, but generally tends to increase compared with ocean water. The most enriched with light molecules ¹H₂¹⁶O water is in the Antarctic (standard light Antarctic precipitation, SLAP). The values of δ deviation for residual heavy isotopes in the water is: δ²H -415.5‰, δ¹⁷O -28.1‰, 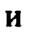 δ¹⁸O -53.9‰, which corresponds to the ¹H₂¹⁶O content at 99.757% [R. van Trigt, Laser Spectrometry for Stable Isotope Analysis of Water Biomedical and Paleoclimatological Applications, 2002, Groningen: University Library Groningen, p. 50].

¹H₂¹⁶O content in natural water ranges from 99.731% 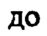 99.757% [Rothman et al., J. Quant. Spectrosc. Radiat. Transfer, 1998, 60, 665. Rothman et al., J. Quant. Spectrosc. Radiat. Transfer, 2003, 82, p.9; R. van Trigt, Laser Spectrometry for Stable Isotope Analysis of Water Biomedical and Paleoclimatological Applications, 2002, Groningen: University Library Groningen, p. 50], 997.0325 g/kg and 997.3179 g/kg of ¹H₂¹⁶O respectively.

Thus, the natural water with content of ¹H₂¹⁶O more than 99.757% (997.3179 g/kg) is not discovered in nature, and the main part of the natural water is water with ¹H₁¹⁶O content of 99.73% (997.0325 g/kg). Weight indices of isotope types of H₂O molecules allow a visual assessment of water purity and homogeneity by isotope composition. In total, weight concentration of water molecules ¹H₂¹⁸O, ¹HD¹⁶O, ¹HD¹⁷O, ¹HD¹⁸O, D₂¹⁶O, D₂¹⁷O, D₂¹⁸O in natural water is up to 2.97 g/kg, which is a significant value comparable to content of other typical components in the natural water. For example, the total salinity of drinking water can be up to 5 g/kg.

According to the claimed invention, using the method and the plant developed by us the content of the lighter constituent of ¹H₂¹⁶O can be commercially increased in the water to the highest level found in nature (SLAP, 99.757%) or more, up to 99.99%. The water resulted from is cleaned both from conventional pollutants and chemical contaminants and from such molecules as: ¹H¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O, which can amount 2.97 g/l and which are a kind of impurities to the main component of water - ¹H₂¹⁶O molecule. As a result, the water becomes almost an isotope homogeneous substance presented with the lighter molecule of water in the amount up to 99.999%, i.e. a light water. This light water is more pure and homogeneous than any chemically pure water with standard qualitative and quantitative isotopic composition. Therefore, it can be called extra pure light water. The more ¹H₂¹⁶O H₂O consists, the more pure and homogeneous in isotopic composition the water is, and thus the inherent properties of water are more stable and definite.

The isotope types of H₂O molecules, whether containing heavy isotopes of D,¹⁷O,¹⁸O or not, influence the basic properties of organic substances, such as proteins, fats, carbohydrates, nucleic acids and some small molecules in different ways (Chervenak et al. JACS, 1994, 116 (23): 10533-10539. Makhatadze et al., Nature Struct. Biol., 1995, 2 (10): 852-855. Connelly et al., PNAS, 1994, 91: 1964-1968. Cupane et al., Nucleic Acids Res. 1980, 8 (18): 4283-4303). The isotope effect of a solvent is a well known phenomenon (Lobyshev V.I., Kalinichenko L.P. "D20 isotope effects in biological systems", Moscow, Science, 1978; Lobyshev V.I. "Mechanisms of D2O thermodynamic and kinetic isotope effects in biological systems", Abstract of a doctoral thesis, Moscow, 1987, Moscow State University, Biological Department), according to an isotopic effect, the presence of isotope types of H₂O water molecules with heavy isotopes reduces the rate of chemical and biochemical reactions. It means that accumulation of heavy isotopes in the human body results in changing normal biochemical processes, which reduces the functional abilities of the body, including the sexual function, depending on a complex set of physiological and psychological factors.

Isotopic composition of a human body is determined by isotopic composition of water and food coming from outside [Nikolaev V. "Prehistoric climatology". "Science and Life", 2001, No. 8]. Therefore human consumption of water and food originally containing more light water substantially facilitates the replacement of heavy isotopes for the light ones. This leads to improvement in functioning of individual systems and the entire human body. In particular, we found that water with higher content of ¹H₂¹⁶O when drinking it instead of water with ordinary content of ¹H₂¹⁶O, has had a positive effect on male potency.

The purpose of this invention is to create the more universal agent compared with the current one without side effects, for use in the treatement of erectile dysfunction.

The description discloses a means to enhance a human sexual activity characterized in that it is a light water containing at least 99.760 molecular % of the lighter ¹H₂¹⁶O molecules and up to 100% of the other forms of water molecules.

In more preferred embodiment the content of ¹H₂¹⁶O in the light water is at least 99.774 molecular % of the total number of water molecules.

Mainly, the means may be used to increase potency, improve erection, and enhance libido.

For this purpose the light water containing at least 99.760 molecular%, or at least 99.774 molecular % of the lighter molecules ¹H₂¹⁶O and up to 100% of the other forms of water molecules, can be a drinking water, distilled water, redistilled water, tridistilled water, deionized water, reverse osmosis water, reagent water, pharmacopoeial purified water, pharmacopoeial water for injection. In other words, efficiency of chemical purification of the light water can depend upon the specific conditions of application.

In the preferred embodiment the light water containing at least 99.760 molecular % or at least 99.774 molecular % of the lighter ¹H₂¹⁶O molecules and up to 100% the other forms of water molecules is used as drinking water, distilled water, pharmacopoeial purified water, pharmacopoeial water for injection.

An assigned task is also solved by the fact that the description discloses a means of enhancing a human sexual activity characterized in that this method includes the step of administrating to a treatable person an effective quantity of the light water containing at least 99.760 molecular % or at least 99.774 molecular % of the lighter ¹H₂¹⁶O molecules and up to 100% the other forms of water molecules.

In the preferred embodiment the content of ¹H₂¹⁶O in the light water is at least 99.760 molecular% of the total number of water molecules.

In the more preferred embodiment of the method content of ¹H₂¹⁶O in the light water is at least 99.774 molecular % of the total number of water molecules. Mainly, the method may be used to increase potency, improve erection, and enhance libido.

Moreover, the light water containing at least 99.760 molecular%, or at least 99.774 molecular % more light molecules ¹H₂¹⁶O and up to 100% of the other forms of water molecules, can be a drinking water, distilled water, redistilled water, tridistilled water, deionized water, reverse osmosis water, reagent water, pharmacopoeial purified water, pharmacopoeial water for injection.

In the preferred embodiment the light water containing at least 99.760 molecular % or at least 99.774 molecular % of the lighter ¹H₂¹⁶O molecules and up to 100% the other forms of water molecules is used as a drinking water, distilled water, pharmacopoeial purified water, pharmacopoeial water for injection.

In the invention, the light water containing at least 99.760 molecular % or at least 99.774 molecular % of the lighter ¹H₂¹⁶O molecules and up to 100% the other forms of water molecules can be administrated orally, parenterally, externally or rectally.

In the preferred embodiment, an effective quantity of the light water containing at least 99.760 molecular % or at least 99.774 molecular % of the lighter 1H216O molecules and up to 100% the other forms of water molecules is administrated orally or parenterally.

In the preferred embodiment, an effective quantity of the light water containing at least 99.760 molecular % or at least 99.774 molecular % of the lighter ¹H₂¹⁶O molecules and up to 100% the other forms of water molecules, is ranging from 0.00625 mg/kg to 37. 5 mg/kg of person weight per day or approximately from 0.5 to 3000 ml for a person per day. An average weight of a person is assumed to be 80 kg.

The use can include an additional step of administrating to a treatable person an effective amount of a preparation to enhance a sexual activity.

Mainly, the preparation for enhancing a human sexual activity can be a medicinal means, an herbal mix, a homeopathic agent, a physiotherapeutic agent, a psychotherapeutic agent, a nutritional agent, a vitamin-mineral additive, a biologically active additive or mixture thereof.

Mainly, the preparation reactant for enhancing the human sexual activity can be vasoactive agent, hormonal agent, hormonal and mimetic agent, an antidepressant, a psychostimulant, a general tonic and restorative agent and/or mixture thereof.

Mainly, the preparation for enhancing a human sexual activity can be in the form of injections, pills, effervescent tablets, powders, oral lozenges, chewing gum or candy, cream, ointments, tinctures, phytoconcentrate, balsams, drops, mixture, plaster.

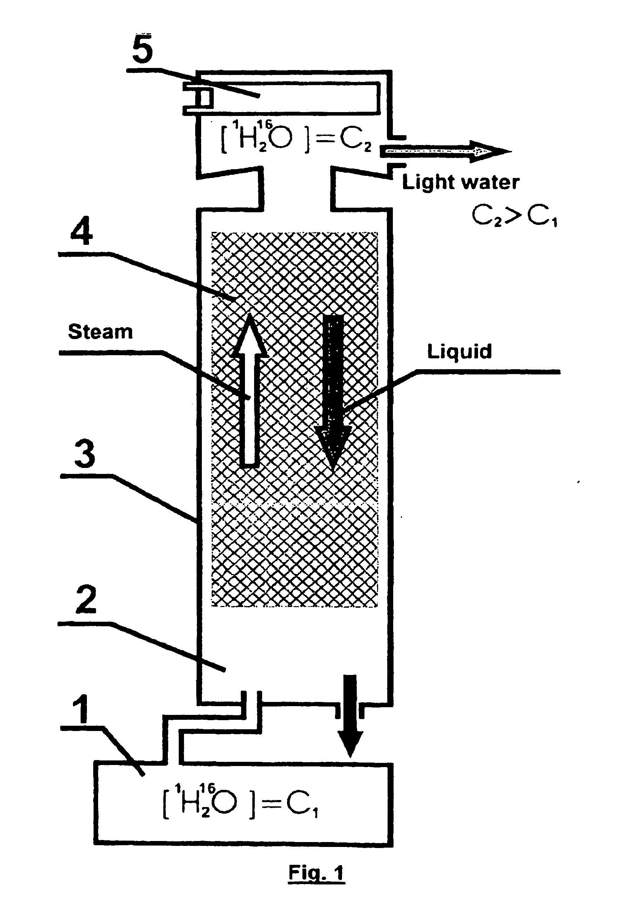

A method and plant were developed to produce, including in production quantities, the extra pure light water used in the invention, containing ¹H₂¹⁶O at least 997,36 g/kg 99.760%, or at least 997,51 g/kg [99.774%] of the total quantity of H₂O. Figure 1 shows a schematic side view of the light water plant.

The plant for production of the extra pure light water includes:
- a steam unit (1) for initial water evaporation with concentration of ¹H₂¹⁶O = C₁;
- a water steam supply unit (2) delivering steam to a rectifying column;
- the rectifying column (3) is a unit of steam-liquid interaction between liquid down flow and steam up flow on a surface of a contact device inside the rectifying column (4), by the backflow of liquid and steam when the liquid main flow and the steam main flow are directed along the axis of the column; the contact device, extending surface of steam-liquid interaction, is either a plate or a structured or randomized nozzle.
- a steam condensing unit (5) with ¹H₂¹⁶O content of C₂ in the condenser installed at the top of the rectifying columns, and accumulation of condensate as condensed extra pure light water at C₂> C₁;

Specified water enrichment level with the lightest constituent of ¹H₂¹⁶O can be obtained by changing, in accordance with the author's papers, number of separation steps in the column, working pressure, as well as ratio of condensate portion selection as water with higher ¹H₂¹⁶O content to the flow of fluid in the rectifying column. The level of enrichment depends upon the specific purpose of the light water.

The molecule of ¹H₂¹⁶O is the lightest one of water molecule isotope types. Therefore the water with increased content of ¹H₂¹⁶O characterized in lower molecular weight, has less density. This water is defined as light purified water or extra pure light water in the claimed invention. In other words, this method provides for a new qualitative level of water purity.

Technical results of the invention is the creation of a physiologically safe means suitable for long-term and unrestricted use by any person, as well as a method of its application for enhancing a human sexual activity by consumption of water with higher content of ¹H₂¹⁶O - the extra pure light water.

Influence of water with the higher content of ¹H₂¹⁶O - the extra pure light water was tested on two groups of volunteers. The first group included men having problems with potency permanently or occasionally. The second group included healthy men who subjectively did not have, in general, problems with potency. Test persons were proposed to drink in specified regimen ordinary water with ordinary content of ¹H₂¹⁶O (control) or the light water (experience), simultaneously assessing their own sexual activity. Work with each test person was carried out individually: quantitative indices of sexual activity (frequency of adequate erection per month) were recorded for each test person prior to the experiment. The test persons did not know what water they were taking throughout the study. Experimental and control water did not differ by the chemistry.

Particular results are shown in the following examples demonstrating the invention. Examples are given for demonstration purposes only.

### Example No 1. Method of the light water production

The light water with higher content of ¹H₂¹⁶O (99.739%) is produced from natural water containing 99.732% of ¹H₂¹⁶O (standard Moscow water) by means of the plant shown on Figure 1. The process of rectification includes natural water evaporation containing 99.732% (C1) of ¹H₂¹⁶O in boiler 1; steam transfer to the lower part 2 of rectifying column 3; contact between the downward fluid flow and upward steam flow on the surface of contact device 4 (for example, plate or nozzle) inside the rectifying column; steam condensation with higher content of ¹H₂¹⁶O (C₂) in steam condensing unit 5 at the top of the column 3; and accumulation of condensate as the light water containing at least 99.739% of ¹H₂¹⁶O molecules (C₂> C₁) of the total number of water molecules.

Production method of water containing at least 99.760 molecular % or at least 99.774 molecular % of ¹H₂¹⁶O molecules of the total number of water molecules is the same. The light water produced is used in examples of this invention.

### Example No 2. Assessment of light water influence on a sexual activity of men with potency disturbances

Each of the 7 volunteers within 30 days was proposed to drink up to one liter (3 - 4 glasses) of ordinary drinking water throughout the day: the first time in the morning obligatory fasting, the second time- the fourth time prior to a meal in the middle of the day and in the evening (no later than 6 p.m.), at the same time noting the level of their sexual activity (frequency of adequate and spontaneous erections per month).

Admission of ordinary drinking water with normal content of ¹H₂¹⁶O in the above regimen had essentially no impact on the level of a sexual activity of test persons, it was similar to previous one. Slight indices increase can be attributed to known "placebo" effect: as the potency to a great extent depends on the psycho-emotional state of the test person, the belief in the effectiveness of a taken agent could contribute to its some real enhancing.

When taking the light water with ¹H₂¹⁶O content equal to 99. 774% in the same drinking regimen (experience) throughout the second month of the studies, each of the test persons noted independently increasing of potency, expressed in the libido enhancement and increase of the erection frequency. The results are shown in Table 1.

In addition, the test persons were given an opportunity to assess subjectively the quality of their sexual life by a five- grade scale. Quality graduation was in the range from 0 - «very bad» to 5 - «very good». The results are also given in Table 1.

**Table 1**

| Group of volunteers | | Initial erection frequency per month | Erection frequency in the control group | Erection frequency in the experimental group ¹H₂ ¹⁶O= 99,774% | Quality of sexual life | |
|---|---|---|---|---|---|---|
| No | Age | | | | Ordinary water | Light water |
| 1 | 64 | 0 | 0 | 4 | 0 | 3,5 |
| 2 | 38 | 1 | 2 | 8 | 2 | 4,5 |
| 3 | 47 | 3 | 3 | 9 | 3 | 4,5 |
| 4 | 40 | 1 | 1 | 6 | 0 | 4 |
| 5 | 51 | 0 | 1 | 5 | 0 | 4 |
| 6 | 42 | 0 | 1 | 7 | 1 | 4 |
| 7 | 53 | 3 | 2 | 9 | 2 | 5 |
| | | | | | | |

In addition to increasing of the adequate erection frequency, the teat persons also noted increasing of spontaneous erection frequency.

### (Reference) Example No 3. Assessment of light water influence on a sexual activity of men without potency disturbance

Each of 5 volunteers (age from 35 to 43 years) within 30 days was proposed to drink up to one liter (3 - 4 glasses) of ordinary drinking water throughout the day: the first time in the morning obligatory fasting, the second time- the fourth time prior to a meal in the middle of the day and in the evening (no later than 6 p.m.), at the same time assessing the level of their sexual activity and quality of their sexual life by five- grade scale.

Just as in the previous case, taking of ordinary drinking water with normal content of ¹H₂¹⁶O in the above regimen had essentially no impact on the level of a sexual activity of test persons.

At the same time, taking the light water with ¹H₂¹⁶O content equal to 99.774% in the same drinking regimen (experience) throughout the second month of the studies with increasing of the frequency of sexual intercourses only slightly, initially quite high in this group of test persons, contributed to libido enhancement and erection intensity increasing against the background of improving the overall tone of the human body. As a result, all test persons assessed the quality of their sexual life as increased to the maximum value: from satisfactory and good to very good.

The presented results show the effectiveness of the light water as a means to normalize the male potency. All tested volunteers (healthy or with relevant problems) had reliable potency increasing: enhancement of libido, increasing of the frequency and intensity of adequate and spontaneous erection, and that was also noted by their sexual partners.

## Claims

1. A water for use in the treatment of erectile dysfunction **characterized in that** it is a light water containing at least 99.760 molecular % of the lighter ¹H₂¹⁶O molecules and made up to 100% with the other forms of water molecules.

2. The water for use of claim 1 **characterized in that** the content of ¹H₂¹⁶O in the light water is at least 99.774 molecular % of total number of water molecules.

3. The water for use of claim 1 or 2 **characterized in that** the light water is chosen from the range: drinking water, distilled water, pharmacopeial water (treated), pharmacopeial water for injections.

4. The water for use of claim 1 wherein the treatment is one in which an effective amount of the light water is to be administrated orally or parenterally.

5. The water for use of claim 1 **characterized in that** an effective amount of the light water is in the range of 0.00625 mg/kg to 37.5 mg/kg of a human weight per day.

6. The water for use of claim 1 **characterized in that** the treatment includes an additional administrating to a treatable person of an effective amount of a preparation for the treatment of erectile dysfunction.

7. The water for use of claim 6 **characterized in that** the additional preparation for the treatment of erectile dysfunction is a medicinal means, a homeopathic agent, a physiotherapeutic agent, a psychotherapeutic agent, a nutritional agent, a vitamin-mineral additive, a biologically active additive and/or mixture thereof.

8. The water for use of claim 7 **characterized in that** the preparation for the treatment of erectile dysfunction is a vasoactive agent, a hormonal agent, a hormonal and mimetic agent, an antidepressant, a psychostimulant, a general tonic and restorative agent and/or mixture thereof.

9. The water for use of claim 7 **characterized in that** the additional preparation for the treatment of erectile dysfunction is in the form of an injection, pill, effervescent tablet, powder, oral lozenges, chewing gum or candy, cream, ointment, tinctures, phytoconcentrate, balsam, drops, mixture, plaster.

## Patentansprüche

1. Wasser zur Verwendung bei der Behandlung von Erektionsstörung **dadurch gekennzeichnet, dass** es ein leichtes Wasser ist, das mindestens 99,760 Molekular-% der leichteren ¹H₂¹⁶O-Moleküle enthält und auf 100% mit den anderen Formen von Wassermolekülen hergestellt wird.

2. Wasser zur Verwendung nach Anspruch 1 **dadurch gekennzeichnet, dass** der Gehalt an ¹H₂¹⁶O im leichten Wasser mindestens 99,774 Molekular-% der Gesamtanzahl an Wassermolekülen ist.

3. Wasser zur Verwendung nach Anspruch 1 oder 2, **dadurch gekennzeichnet, dass** das leichte Wasser aus der Reihe: Trinkwasser, destilliertes Wasser, Arzneiwasser (pharmacopeial water) (behandelt), Arzneiwasser für Injektionen ausgewählt ist.

4. Wasser zur Verwendung nach Anspruch 1, wobei die Behandlung eine ist, bei der eine effektive Menge des leichten Wassers oral oder parenteral verabreicht wird.

5. Wasser zur Verwendung nach Anspruch 1 **dadurch gekennzeichnet, dass** eine effektive Menge des leichten Wassers im Bereich von 0,00625 mg/kg bis 37,5 mg/kg eines menschlichen Gewichts pro Tag liegt.

6. Wasser zur Verwendung nach Anspruch 1 **dadurch gekennzeichnet, dass** die Behandlung eine zusätzliche Verabreichung einer effektiven Menge einer Zubereitung für die Behandlung von Erektionsstörung an eine behandelbare Person einschließt.

7. Wasser zur Verwendung nach Anspruch 6 **dadurch gekennzeichnet, dass** die zusätzliche Zubereitung für die Behandlung von Erektionsstörung ein medizinisches Mittel, ein homöopathisches Mittel, ein physiotherapeutisches Mittel, ein psychotherapeutisches Mittel, ein Nahrungsmittel, ein Vitamin-Mineral-Zusatzstoff, ein biologisch aktiver Zusatzstoff und/oder eine Mischung davon ist.

8. Wasser zur Verwendung nach Anspruch 7 **dadurch gekennzeichnet, dass** die Zubereitung für die Behandlung von Erektionsstörung ein vasoaktives Mittel, ein hormonales Mittel, ein hormonales und mimetisches Mittel, ein Antidepressivum, ein Psychostimulans, ein allgemeines tonisches und restauratives Mittel und/oder eine Mischung davon ist.

9. Wasser zur Verwendung nach Anspruch 7 **dadurch gekennzeichnet, dass** die zusätzliche Zubereitung für die Behandlung von Erektionsstörung in Form einer Injektion, Pille, Brausetablette, Pulvers, oraler Lutschtabletten, Kaugummis oder Bonbons, Creme, Salbe, Tinkturen, Phytokonzentrats, Balsams, Drops, Mischung, Pflasters ist.

## Revendications

1. Eau pour une utilisation dans le traitement d'un dysfonctionnement de l'érection, **caractérisée en ce qu'**il s'agit d'eau légère contenant un pourcentage moléculaire d'au moins 99,760 % des molécules de ¹H₂¹⁶O plus légères et la proportion restante, pour parvenir à 100 %, des autres formes de molécules d'eau.

2. Eau pour une utilisation suivant la revendication 1, **caractérisée en ce que** la quantité de ¹H₂¹⁶O dans l'eau légère est un pourcentage moléculaire d'au moins 99,774 % du nombre total des molécules d'eau.

3. Eau pour une utilisation suivant la revendication 1 ou 2, **caractérisée en ce que** l'eau légère est choisie dans l'ensemble suivant : l'eau potable, l'eau distillée, l'eau de la pharmacopée (traitée), l'eau de la pharmacopée pour injections.

4. Eau pour une utilisation suivant la revendication 1, le traitement étant un traitement dans lequel une quantité efficace de l'eau légère est destinée à être administrée par voie orale ou parentérale.

5. Eau pour une utilisation suivant la revendication 1, **caractérisée en ce qu'**une quantité efficace de l'eau légère est comprise dans l'intervalle de 0,00625 mg/kg à 37,5 mg/kg de poids humain par jour.

6. Eau pour une utilisation suivant la revendication 1, **caractérisée en ce que** le traitement comprend une administration supplémentaire à un sujet apte au traitement d'une quantité efficace d'une préparation pour le traitement d'un dysfonctionnement de l'érection.

7. Eau pour une utilisation suivant la revendication 6, **caractérisée en ce que** la préparation supplémentaire pour le traitement d'un dysfonctionnement de l'érection est un moyen médicinal, un agent homéopathique, un agent physiothérapeutique, un agent psychothérapeutique, un agent nutritionnel, un additif à base de vitamines - substances minérales, un additif biologiquement actif et/ou un de leurs mélanges.

8. Eau pour une utilisation suivant la revendication 7, **caractérisée en ce que** la préparation pour le traitement d'un dysfonctionnement de l'érection est un agent vaso-actif, un agent hormonal, un agent hormonal et mimétique, un antidépresseur, un psychostimulant, un tonique générale et de reconstitution et/ou un de leurs mélanges.

9. Eau pour une utilisation suivant la revendication 7, **caractérisée en ce que** la préparation supplémentaire pour le traitement d'un dysfonctionnement de l'érection est sous forme d'une préparation injectable, d'une pilule, d'un comprimé effervescent, d'une poudre, de tablettes buccales, d'une gomme à mâcher ou d'un produit de confiserie, d'une crème, d'une pommade, de teintures, d'un phytoconcentré, d'un baume, de gouttes, d'un mélange ou d'un emplâtre.
